# EUROPÄISCHE PATENTSCHRIFT

(11) **EP 3 296 387 B1**
(45) Veröffentlichungstag und Bekanntmachung des Hinweises auf die Patenterteilung: **15.05.2019**
(21) Anmeldenummer: 16189105.6
(22) Anmeldetag: 16.09.2016
(51) Int. Cl.: C12M 1/34, C12M 1/36

(54) **VERFAHREN ZUR ÜBERWACHUNG VON BIOPROZESSEN**
METHOD FOR MONITORING BIO PROCESSES
PROCEDE DE SURVEILLANCE DE BIOPROCESSUS

(43) Veröffentlichungstag der Anmeldung: 21.03.2018
(73) Patentinhaber: Siemens Aktiengesellschaft, 80333 München (DE)
(72) Erfinder: Scheiblauer, Johannes, 3364 Neuhofen/Ybbs (AT); Joksch, Martin, 3423 St.Andrä-Wördern (AT)
(74) Vertreter: Maier, Daniel Oliver

(56) Entgegenhaltungen:
- EP-A2- 2 530 629
- DE-A1- 10 141 556
- DE-A1- 10 141 557
- DE-A1-102007 047 175
- US-A1- 2015 302 318
- CHARBONNIER S ET AL: "A self-tuning adaptive trend extraction method for process monitoring and diagnosis", JOURNAL OF PROCESS CONTROL, OXFORD, GB, Bd. 22, Nr. 6, 27. März 2012 (2012-03-27), Seiten 1127-1138, XP028520057, ISSN: 0959-1524, DOI: 10.1016/J.JPROCONT.2012.03.010 [gefunden am 2012-04-04]
- JIN HUAIPING ET AL: "Multi-model adaptive soft sensor modeling method using local learning and online support vector regression for nonlinear time-variant batch processes", CHEMICAL ENGINEERING SCIENCE, Bd. 131, 1. April 2015 (2015-04-01), - 1. April 2015 (2015-04-01), Seiten 282-303, XP029236116, ISSN: 0009-2509, DOI: 10.1016/J.CES.2015.03.038

## Beschreibung

### Technisches Gebiet

Die gegenständliche Erfindung betrifft allgemein das Gebiet der Biotechnologie und des so genannten Bio-Engineerings. Im Speziellen bezieht sich die vorliegende Erfindung auf ein Verfahren zur Überwachung von Bioprozessen, insbesondere von Fermentationsprozessen. Dabei wird ein Verlauf eines Bioprozesses, insbesondere eines Fermentationsprozesses, mittels eines Prozessmodells vorhergesagt und während des Verlaufs des Bioprozesses Schätzwerte für wichtige, biologische Prozessparameter mit Hilfe des Prozessmodells abgeschätzt.

### Stand der Technik

Bioprozesse, bei welchen lebende Systeme wie z.B. Zellen, Organismen, Pilze, Bakterien, etc. für eine Produktion von Stoffen verwendet werden, werden in verschiedenen industriellen Bereichen (z.B. Lebensmittel- und Getränkeindustrie, biopharmazeutische Industrie, Herstellung von Biokraftstoff, etc.) eingesetzt. Insbesondere Fermentationsprozesse stellen eine Schlüsseltechnik z.B. bei der Erzeugung von Bier, Whisky oder Wein, bei der Erzeugung von Biokraftstoff oder bei der Herstellung von Impfstoffen oder Antibiotika dar. Unter Fermentation oder Fermentierung wird in der Biotechnologie üblicherweise eine enzymatische Umwandlung organischer Ausgangsstoffe - eines Substrats wie z.B. Zucker, Glukose, etc. - in z.B. Säuren, Gase oder Alkohol verstanden. Bioprozesse, wie eine Fermentation, werden bewusst durch Zugabe von Bakterien-, Pilz- oder sonstigen biologischen Zellkulturen - der sogenannten Biomasse - zu einem Substrat ausgelöst.

Bioprozesse laufen üblicherweise in so genannten Bioreaktoren ab, in welchen die Umgebungs- und Reaktionsbedingungen gesteuert und optimiert werden können. Für Fermentationsprozesse werden die Bioreaktoren auch als Fermenter bezeichnet. Auf diese Weise kann von der verwendeten Biomasse der gewünschte Stoff unter möglichst optimalen Bedingungen bzw. in einer gewünschten Konzentration produziert werden. Im Bioreaktor können dazu verschiedene Umgebungs- und/oder Prozessparameter für den jeweiligen Bioprozess wie z.B. pH-Wert, Temperatur, Sauerstoffzufuhr, Stickstoffzufuhr, Glukosegehalt oder Rühreinstellungen, etc. geregelt und gesteuert werden. Bioprozesse sind allerdings biologisch komplex und sehr empfindlich. Um die entsprechenden Umgebungsbedingungen im Bioreaktor für einen konsistenten und optimalen Verlauf des Bioprozesses sicher zu stellen und damit die Biomasse bzw. die verwendeten bioaktiven Zellen in der Nährlösung wachsen und den gewünschten Stoff produzieren können, ist eine laufende genaue Überwachung des Bioprozesses notwendig.

Einen wichtigen Eckpfeiler in der Überwachung von Bioprozessen, insbesondere Fermentationsprozessen, stellt beispielsweise eine mathematische Modellierung dar, bei welcher ein mathematisches Prozessmodell für den jeweiligen Bioprozess oder Fermentationsprozess entwickelt wird. Mit Hilfe dieses Prozessmodells kann beispielsweise ein Verlauf des Bioprozesses oder der Fermentation vorhergesagt werden. Weiterhin können mit dem Prozessmodell nicht direkt messbare Größen des Bioprozesses - so genannte Soft Sensors - sowie wichtige Prozessparameter (z.B. eine Konzentration der Biomasse, der Glukose, des produzierten Stoffes, des gelösten Sauerstoffs, etc., ein Fermentervolumen, usw.) mathematisch für jeden Zeitabschnitt des gesamten Prozessverlaufs abgeschätzt werden.

Bei der mathematischen Modellierung von Bioprozessen können üblicherweise zwei Gruppen von Modellierungsansätzen unterschieden werden: der so genannte deterministische Ansatz und der so genannte statistische Ansatz. Beim deterministischen Ansatz bzw. bei einem deterministischen Prozessmodell werden bekannte, biochemische Vorgänge innerhalb und außerhalb der bioaktiven Zellen des Bioprozesses mittels mathematischer Gleichungen wie z.B. Differentialgleichungen beschrieben. Aus der Schrift: Sonnleitner, B. und Käppeli, O. (1986); Growth of S. cerevisiae is controlled by its limited respiratory capacity: Formulation and verification of a hypothesis. Biotechnology and Bioengineering, Vol. 28: Pp. 927-937. ist beispielsweise ein deterministisches Prozessmodell bekannt, mit welchem fundamentale Stoffwechselpfade des Mikroorganismus Saccharomyces cerevisiae bzw. S. cerevisiae (deutsch: Backhefe) mathematisch beschrieben werden.

Beim statischen Ansatz für die mathematische Modellierung von Prozessmodellen werden meist historische bzw. statische Daten des jeweiligen Bioprozesses mathematisch verarbeitet, wobei bei Prozessmodellen nach dem statischen Ansatz häufig ein deterministisches Prozessmodell mit statistischen bzw. stochastischen Ansätzen kombiniert wird. Die eher komplexen, statischen Modellansätze wurden erst mit steigender Rechnerleistung praktikabel. Die Vorhersage des jeweiligen Prozessverlaufs bzw. die Abschätzung von Prozessparametern kann beispielsweise mittels neuronaler Netze - wie z.B. in den Schriften: Karakuzu, C. et al. (2006); Modelling, on-line state estimation and fuzzy control of production scale fedbatch baker's yeast fermentation. Control Engineering Practice, 14: 959-974. oder Nagy, Z.K. (2007); Model based control of a yeast fermentation bioreactor using optimally designed artificial neural networks. Chemical Engineering Journal 127 (1): 95-109. beschrieben - oder unter Einsatz des so genannten "Golden-Batch"-Modells - wie z.B. in der Schrift: Besenhard, M. O. et al. (2016); Multivariate Process Control of a Small Scale Bioreactor in a PAT Environment. Journal of Intelligent Manufacturing, Pp. 1-14. beschrieben - durchgeführt werden.

Für die Überwachung eines Bioprozesses kann beispielsweise eine mit dem entsprechenden Prozessmodell erstellte Vorhersage mit Messungen, welche während des Verlaufs des Bioprozesses durchgeführt werden, verglichen werden. Bei Abweichungen kann ein Eingreifen durch einen Operator oder - bei einer entsprechenden Regelung - z.B. durch das Prozessmodell selbst eingeleitet werden. Aus der Schrift EP 2 530 629 A2 ist beispielsweise eine Methode zum Modellieren und Simulieren eines Fermentationsprozesses zum Brauen von Bier bekannt. Dabei wird zu einem ersten Zeitpunkt eine Konzentration von zumindest einem Substrat des Fermentationsprozesses bestimmt. Für einen späteren, zweiten Zeitpunkt wird mit Hilfe des Prozessmodells die Konzentration des zumindest einen Substrats des Fermentationsprozesses vorhergesagt. Ist eine Differenz zwischen dem mit dem Prozessmodell vorhergesagten Wert und einem Zielwert für Konzentration des zumindest einen Substrats des Fermentationsprozesses größer als ein vorgegebener Schwellwert, so wird über ein Kontrollsystem in den Fermentationsprozess eingegriffen und zumindest ein Betriebsparameter des Fermentationsprozesses angepasst.

Dabei bleibt allerdings unberücksichtigt, dass Bioprozesse trotz gleicher Voraussetzungen bei Wiederholungen variieren und damit trotz Abweichungen vom entsprechenden Prozessmodell Produkte mit guter Qualität liefern können. Eine Anpassung des laufenden Bioprozesses an das Prozessmodell durch ein Eingreifen kann daher nicht bei jeder festgestellten Abweichung notwendig sein. Für festgestellte Abweichung können unterschiedliche Ursachen vorliegen wie z.B. Fehler im Prozess (z.B. Ausfall von Regelungsmechanismen oder Sensoren beim Bioreaktor, Verunreinigungen, falsche Begasung, etc.), biologische Variabilität (z.B. Unterschiede in der Vorkultur der verwendeten Biomasse; Unterschiede im Zellstamm, etc.) oder Variabilität in der Prozessführung (z.B. Unterschiede bei den Rohstoffen, Schwankungen in der Substratzusammensetzung, Unterschiede bei der technischen Ausrüstung). Je nach der Ursache der festgestellten Abweichung kann ein Eingreifen in den Bioprozess wie z.B. bei Prozessfehlern notwendig sein, um beispielsweise Schäden am Equipment, etc. zu verhindern oder um im schlimmsten Fall den Bioprozess abzubrechen. Festgestellte Abweichungen, die beispielswiese aufgrund von biologischer Variabilität oder Variabilität der Prozessführung zustande kommen, sind meist der Produktqualität nicht abträglich und bedürfen daher keines Eingriffs. Üblicherweise kann anhand des Prozessmodells die unterschiedliche Ursache für die jeweils festgestellte Abweichung zwischen modellbasierter Vorhersage und tatsächlich ablaufendem Prozess nicht eruiert werden, da die Abweichungen auf Basis des Prozessmodells nicht bewertet werden können. Aus der festgestellten Abweichung geht damit nicht hervor, ob die Abweichung nur einer biologischen Variabilität oder Variabilität der Prozessführung geschuldet ist, oder ob es sich um einen ernsthaften Prozessfehler handelt. Dadurch ist einerseits die Qualität der Vorhersage durch das Prozessmodell unklar und andererseits kann es im Zweifel zu möglicherweise unnötigen Eingriffen und/oder Abbrüchen von Bioprozessen sowie zusätzlichen Kosten kommen.

### Darstellung der Erfindung

Der Erfindung liegt daher die Aufgabe zugrunde, ein Verfahren zur Überwachung von Bioprozessen anzugeben, bei welchem eine Vorhersagequalität eines zur Vorhersage eines Bioprozesses eingesetzten Prozessmodells auf einfache Weise bewertet und gesteigert werden kann und festgestellte Abweichungen zwischen dem Prozessmodell und dem Verlauf des Bioprozesses entsprechend qualitativ bewertet werden können.

Diese Aufgabe wird durch ein Verfahren der eingangs genannten Art mit den Merkmalen des unabhängigen Patentanspruchs gelöst. Vorteilhafte Ausführungsformen der vorliegenden Erfindung sind in den abhängigen Ansprüchen beschrieben.

Der Hauptaspekt der erfindungsgemäß vorgeschlagenen Lösung besteht darin, dass durch eine Variation eines definierten Modellparameters des Prozessmodells im Falle von Abweichungen zwischen dem tatsächlichen Prozessverlauf und der Vorhersage auf Basis des Prozessmodells das Prozessmodell auf einfache Weise an den tatsächlichen Prozessverlauf einfach und rasch angepasst werden kann. Damit können beispielsweise bessere Schätzwerte für den ausgewählten Prozessparameter, aber auch für die weiteren Prozessparameter, welche mit dem Prozessmodell abgeschätzt werden, gewonnen werden. Die Qualität der Vorhersage des Bioprozesses durch das Prozessmodell kann damit auf einfache Weise qualitativ gesteigert werden. Weiterhin können auftretende Abweichungen durch das iterative, erfindungsgemäße Verfahren sehr rasch und einfach qualitativ eingestuft werden. Es kann beispielsweise auf einfache Weise zwischen biologischer Variabilität bzw. Variabilität in der Prozessführung (z.B. Unterschiede in der Vorkultur oder im verwendeten Zellstamm, Unterschiede beim Rohstoff bzw. Substrat für den Bioprozess) und Fehlern im Bioprozess selbst (z.B. falsche Begasung, fehlerhafte Regelung des pH-Werts, etc.) unterschieden werden. Wird eine Abweichung als Fehler im Bioprozess bewertet, kann rasch von einem Operator eingegriffen werden, um beispielsweise Schäden am Equipment oder gegebenenfalls einen Abbruch des Bioprozesses zu verhindern. Es kann mit dem erfindungsgemäßen Verfahren auf einfache Weise eruiert werden, aus welchem Grund die Abweichung im Prozess auftritt, und ob ein Eingriff in den Verlauf des Bioprozesses notwendig ist. Unnötige Eingriffe in den Bioprozess oder nicht notwendige Abbrüche können damit durch das erfindungsgemäße Verfahren verhindert bzw. zumindest reduziert werden.

Erfindungsgemäss wird als definierter Modellparameter, welcher im Prozessmodell eingesetzt wird, ein so genannter Verzögerungsterm verwendet. Bei Überschreiten des Schwellwertes durch die Abweichung zwischen dem jeweiligen Messwert und dem entsprechenden Schätzwert für den zumindest einen ausgewählten Prozessparameter wird der Verzögerungsterm innerhalb vorgegebener Grenzwerte verändert. Die Grenzwerte, innerhalb welcher der Verzögerungsterm variiert wird, werden idealerweise durch eine im Bioprozess verwendete Biomasse, insbesondere durch die eingesetzten bioaktiven Zellen oder Organismen, vorgegeben. Je nach der jeweils eingesetzten Biomasse - z.B. Zellstamm oder für die verwendete Biomasse eingesetzte Vorkultur - kann ein Bioprozess etwas schneller oder langsamer ablaufen. Im Prozessmodell wird dies durch den so genannten Verzögerungsterm oder "Lag Term" berücksichtigt. Durch eine Variation des Verzögerungsterms innerhalb biologisch sinnvoller Grenzwerte kann im Schritt d sehr einfach das Prozessmodell verändert bzw. an eine mögliche vorhandene, biologische oder Prozess bedingte Variabilität angepasst werden.

Kann bei einer vorgegebenen Anzahl an Wiederholungen der Schritte b bis d die Abweichung zwischen dem gemessenen Wert und dem Schätzwert für den zumindest einen ausgewählten Prozessparameter nicht behoben werden - d.h. der Schwellwert wird trotz Variation des Verzögerungsterms weiterhin überschritten, so kann die Abweichung als Prozessabweichung bzw. als Fehler im Prozess bewertet werden. Es kann dann beispielsweise zumindest eine Warnung an den Operator gesendet werden, um z.B. den Prozess zu begutachten, die Sensorik zu überprüfen, etc. Weiterhin kann daraus abgeleitet werden, dass die Schätzwerte für die weiteren Prozessparameter sowie für nicht direkt messbare Größen - so genannte Soft Sensors - nicht valide sind.

Lässt sich die Abweichung zwischen dem gemessenen Wert und dem Schätzwert für den zumindest einen ausgewählten Prozessparameter durch die Variation des Verzögerungsterms innerhalb der vorgegebenen Anzahl an Wiederholungen der Schritte b bis d beheben, so kann die Abweichung beispielsweise als eine biologische Variabilität bedingt durch z.B. Unterschied in der eingesetzten Vorkultur oder Zellkultur oder im eingesetzten Zellstamm eingestuft werden. Der Bioprozess kann damit weiter ablaufen. Die mit dem Prozessmodell vorhergesagten, weiteren Prozessparameter können als valide eingestuft werden.

Es ist günstig, wenn als zu messender Prozessparameter ein Prozessparameter des Bioprozesses ausgewählt wird, für welchen im Verlauf des Bioprozesses Messwerte annähernd in Echtzeit bestimmt werden können. Damit kann die Abweichung zwischen dem Prozessmodell und dem Verlauf des Bioprozesses annähernd in Echtzeit bestimmt werden, insbesondere wenn das Prozessmodell parallel zum laufenden Bioprozess berechnet wird. Es kann damit ein direkter Vergleich zwischen dem gemessenen Wert des gewählten Prozessparameters und dem vom Prozessmodell berechneten bzw. vorhergesagten Schätzwert für den Prozessparameter durchgeführt werden. Damit wird auch bei Vorliegen eines Fehlers im Bioprozess ein zeitnahes Eingreifen ermöglicht.

Zweckmäßigerweise werden die Wiederholungen der Schritte b bis d des erfindungsgemäßen Verfahrens - d.h. Vergleich zwischen Messwert und Schätzwert des gewählten Prozessparameters; Vergleichen der Abweichung mit dem vorgegebenen Schwellwert und Verändern des Modellparameters des Prozessmodells bei Überschreiten des Schwellwerts - mit dem jeweils veränderten Modellparameter in zeitlich geringen Abständen wie z.B. alle 10 Sekunden durchgeführt. Damit kann das Prozessmodell z.B. bei Vorliegen von biologischer Variabilität oder Variabilität in der Prozessführung (z.B. Unterschiede bei den Rohstoffen, Schwankungen bei der Zusammensetzung des Substrats, etc.) rasch angepasst werden, damit eine entsprechend valide Vorhersage des Bioprozesses für den weiteren Prozessverlauf durchgeführt werden kann. Bei Fehlern im Bioprozess kann zeitnahe reagiert werden. Durch ein rasches Eingreifen können beispielsweise Schäden am Bioprozess und/oder am Equipment vermieden bzw. verhindert werden.

Bei der Ausgestaltung der Erfindung ist vorgesehen, dass als Prozessparameter, welcher während des Verlaufs des Bioprozesses gemessen wird, zumindest der so genannte respiratorische Quotient - kurz auch als RQ bezeichnet - ausgewählt wird. Der respiratorische Quotient ist ein Prozessparameter bei Bioprozessen, insbesondere bei Fermentationsprozessen, welcher einen Indikator für die Vorgänge innerhalb einer bioaktiven Zelle darstellt. Der respiratorische Quotient beschreibt ein Verhältnis der in einer bestimmten Zeit produzierten Kohlenstoffdioxidmenge (CO₂) zum gleichzeitig verbrauchten Sauerstoff (O₂). Während des Verlaufs eines Bioprozess kann der respiratorische Quotient sehr einfach in Echtzeit z.B. mittels der so genannten Off-Gas-Analyse gemessen werden.

Zusätzlich, können als Prozessparameter, welche während des Verlaufs des Bioprozesses gemessen werden, auch eine Konzentration der Biomasse und/oder eine Konzentration des Substrats ausgewählt werden. Die Bestimmung der aktuellen Messwerte der Konzentration der Biomasse während des Verlaufs des Bioprozesses kann beispielsweise auf Basis der elektrischen Eigenschaften der Biomasse erfolgen. Aktuelle Messwerte für die Konzentration des Substrats (z.B. Glukose, etc.) können ebenfalls im Verlauf des Bioprozesses z.B. anhand von spektroskopischen Eigenschaften mittels Spektroskopie, insbesondere mittels Reflexionsspektroskopie, bestimmt werden.

Weiterhin ist es vorteilhaft, wenn zum Abschätzen der Prozessparameter des Bioprozesses ein so genanntes deterministisches Prozessmodell eingesetzt wird. Bei einem deterministischen Ansatz für eine Abbildung eines Bioprozesses als Modell wird das Wissen über die jeweiligen prozessspezifischen, biochemischen Vorgänge innerhalb und außerhalb der eingesetzten Biomasse bzw. Zellen während des Ablaufs des Bioprozesses in mathematische Gleichungen übersetzt.

### Kurzbeschreibung der Zeichnung

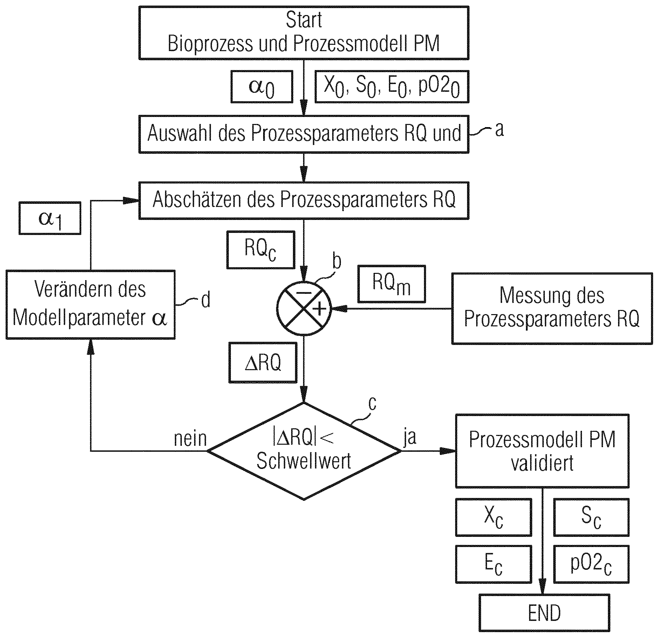

Die Erfindung wird nachfolgend in beispielhafter Weise anhand der beigefügten Figur 1 erläutert. Figur 1 zeigt schematisch einen beispielhaften Ablauf des erfindungsgemäßen Verfahrens zur Überwachung von Bioprozessen, deren Verlauf mit Hilfe mit einem Prozessmodell vorhergesagt wird.

### Ausführung der Erfindung

Figur 1 zeigt schematisch einen beispielshaften Ablauf des erfindungsgemäßen Verfahrens zur Überwachung von Bioprozessen anhand eines beispielhaften Bio- bzw. Fermentationsprozesses mit Backhefe bzw. S. cerevisiae als Biomasse X. Als Biomasse X können in Bioprozesse allerdings auch andere bioaktive Organismen (Pilze, Bakterien, etc.) wie z.B. E. coli, Schimmelpilze, etc. eingesetzt werden. Der Bio- oder Fermentationsprozess läuft üblicherweise in einem Bioreaktor ab, in welchem die Umgebungs- und Reaktionsbedingungen für einen Bioprozess wie eine Fermentation mit Backhefe optimiert und gesteuert werden können. Dazu werden im Bioreaktor während des Bioprozesses verschiedene Umgebungs- und/oder Prozessparameter X, S, E, pO2, RQ geregelt und/oder gemessen wie z.B. pH-Wert, Temperatur, Sauerstoffzufuhr, Stickstoffzufuhr, Rühreinstellungen, Glukosekonzentration S, Konzentration der Biomasse X oder des zu produzierenden Stoffes E (z.B. Ethanol), Konzentration von gelöstem Sauerstoff pO2 oder der so genannte respiratorische Quotient RQ, etc.

Für eine Erhaltung und Regelung der Umgebungs- und Reaktionsbedingungen ist eine Überwachung des Bioprozesses notwendig. Dazu wird für den Ablauf des Bioprozesses ein Prozessmodell PM erstellt, mit welchem während des Verlaufs des Bioprozesses für jeden Zeitabschnitt Schätzwerte X_{c}, S_{c}, E_{c}, pO2_{c}, RQ_{c} für die jeweiligen biologischen Prozessparameter X, S, E, pO2, RQ, welche beispielsweise für eine Überwachung und Regelung relevant sind, abgeschätzt oder berechnet werden können. Bei einem Fermentationsprozess mit Backhefe können beispielsweise eine Konzentration von Biomasse X, Glukose S, Ethanols E, gelösten Sauerstoffs pO2 und der so genannten respiratorischen Quotienten RQ mit den Prozessmodell PM auf Basis von Anfangswerten X₀, S₀, E₀, pO2₀ für die Überwachung ermittelt werden. Je nach dem jeweiligen Bioprozess können mit Hilfe des jeweiligen Prozessmodell PM auch weitere und/oder andere Prozessparameter abgeschätzt werden. Als Anfangswerte X₀, S₀, E₀, pO2₀ der Prozessparameter X, S, E, pO2 für das Prozessmodell PM werden üblicherweise entsprechenden Anfangswerte der Prozessparameter X, S, E, pO2 des realen Bioprozesses verwendet.

Zum Abschätzen der Prozessparameter X, S, E, pO2, RQ für den jeweiligen Zeitabschnitt des Bioprozesses kann beispielsweise ein deterministisches Prozessmodell PM herangezogen werden, von welchem das Wissen über die jeweiligen prozessspezifischen, biochemischen Vorgänge innerhalb und außerhalb der eingesetzten Biomasse bzw. Zellen während des Ablaufs des Bioprozesses in mathematische Gleichungen übersetzt wird. Weiterhin kann das Prozessmodell PM zumindest einen definierten Modellparameter α mit einem Anfangswert α₀ enthalten. Dieser Modellparameter α kann beispielsweise ein so genannter Verzögerungsterm α oder "Lag-Term" sein, von welchem berücksichtigt wird, dass je nach Operator, Zellstamm der Biomasse X, Vorkultur der Biomasse X, aufgrund von Schwankungen beim Substrat S, etc. - insbesondere trotz sonst gleicher Bedingungen - der Bioprozess bzw. die Fermentation mittels Backhefe schneller oder langsamer ablaufen kann.

Das erfindungsgemäße Verfahren beginnt mit einem Startschritt, bei welchem der Bioprozess bzw. die Fermentation mit Backhefe im Bioreaktor beispielsweise durch Zugabe der Biomasse X zum Substrat bzw. Glukose S gestartet wird. Parallel dazu wird mit dem Startschritt auch die Berechnung bzw. Abschätzung der Prozessparameter X, S, E, pO2, RQ für jeden Zeitabschnitt des Bioprozesses auf Basis der Anfangswerte X₀, S₀, E₀, pO2₀ der Prozessparameter X, S, E, pO2 durch das Prozessmodell PM gestartet. Bioprozess bzw. Fermentation und Prozessmodell soll weitgehend parallel ablaufen. Beim Startschritt wird im Prozessmodell PM der Anfangswert α₀ des Modellparameter α bzw. Verzögerungsterms α eingesetzt und die Prozessparameter X, S, E, pO2, RQ entsprechend abgeschätzt.

In einem ersten Verfahrensschritt a wird zumindest ein Prozessparameter RQ des Bioprozesses ausgewählt. Für diesen ausgewählten Prozessparameter RQ werden während des Verlaufs des Bioprozesses aktuelle Messwerte RQₘ bestimmt. Idealerweise können die Messwerte RQₘ des gewählten Prozessparameters RQ während des Ablaufs des Bioprozesses annähernd in Echtzeit bestimmt werden. Im vorliegenden beispielhaften Verfahrensverlauf für eine Fermentation mit Backhefe wurde beispielsweise der so genannter respiratorische Quotient RQ als zu messender Prozessparameter RQₘ ausgewählt. Der respiratorische Quotient RQ beschreibt ein Verhältnis der in einer bestimmten Zeit produzierten Kohlenstoffdioxidmenge (CO₂) zum gleichzeitig verbrauchten Sauerstoff (O₂) und stellt einen Indikator für Vorgänge innerhalb einer bioaktiven Zelle dar. Der Prozessparameter RQ kann relativ einfach z.B. mittels der so genannten Off-Gas-Analyse in Echtzeit während des Bioprozesses bzw. während der Fermentation gemessen werden.

Zusätzlich, kann im ersten Verfahrensschritt a auch eine Konzentration der Biomasse X und/oder eine Konzentration der Glukose S beim beispielhaften Bioprozess - Fermentation mit Backhefe - als zu messender Prozessparameter Xₘ, Sₘ - gewählt werden. Die jeweilige Konzentration der Biomasse X ist beispielsweise anhand der elektrischen Eigenschaften der Zellen bzw. der Biomasse X während des Ablaufs der Fermentation messbar. Die jeweilige Konzentration der Glukose S kann z.B. mit Hilfe von Spektroskopie, insbesondere Reflexionsspektroskopie, anhand von spektroskopischen Eigenschafte des Substrats während des Ablaufs der Fermentation aktuell bestimmt werden.

In einem zweiten Verfahrensschritt b wird dann ein aktueller Messwert RQₘ des ausgewählten Prozessparameters RQ mit dem entsprechenden vom Prozessmodell PM abschätzten Schätzwert RQ_{c} des gewählten Prozessparameters RQ verglichen. Dies kann bei einem Parallel-Laufen von Bioprozess und Prozessmodell in annähernder Echtzeit durchgeführt werden. D.h. es werden ein aktueller Messwert RQₘ für den respiratorischen Quotienten RQ und ein für den Zeitpunkt der Messung vom Prozessmodell PM bestimmter Schätzwert RQ_{c} für den respiratorischen Quotienten RQ während des Ablaufs der Fermentation möglichst zeitgleich ermittelt und dann miteinander verglichen. Der Vergleich kann beispielsweise als Subtraktion des Schätzwertes RQ_{c} vom Messwert RQₘ oder als Subtraktion des Messwertes RQₘ vom Schätzwert RQ_{c} ausgeführt sein. Sind der Messwert RQₘ und der Schätzwert RQ_{c} für den jeweiligen Zeitpunkt nicht ident, so wird von der Subtraktion eine Abweichung ΔRQ geliefert.

In einem dritten Verfahrensschritt c wird die Abweichung ΔRQ zwischen dem jeweiligen aktuellen Messwert RQₘ und dem entsprechenden Schätzwert RQ_{c} mit einem vorgegebenen Schwellwert verglichen. Da die Abweichung ΔRQ zwischen Messwert RQₘ und Schätzwert RQ_{c} aufgrund des Vergleichs mittels Subtraktion positives oder negatives Vorzeichen aufweisen kann, wird für den Vergleich mit dem vorgegebenen Schwellwert ein Betrag der im zweiten Verfahrensschritt b ermittelten Abweichung ΔRQ verwendet.

Wird im dritten Verfahrensschritt c festgestellt, dass die Abweichung ΔRQ bzw. der Betrag der Abweichung ΔRQ kleiner als der vorgegebene Schwellwert ist, so wird das Prozessmodell PM als gute Vorhersage des Ablaufs des Bioprozesses bzw. der Fermentation eingestuft. D.h. es wird davon ausgegangen, dass vom Prozessmodell PM valide Schätzwerte X_{c}, S_{c}, E_{c}, pO2_{c} der weiteren Prozessmeter X, S, E, pO2 geliefert werden und diese Schätzwerte X_{c}, S_{c}, E_{c}, pO2_{c} im Verlauf des Bioprozesses bzw. der Fermentation für eine Überwachung herangezogen werden können. Das erfindungsgemäße Verfahren wird mit der Validierung des genutzten Prozessmodells PM beendet.

Wird im dritten Verfahrensschritt c festgestellt, dass die Abweichung ΔRQ bzw. der Betrag der Abweichung ΔRQ den vorgegebenen Schwellwert überschritten hat, so wird ein vierter Verfahrensschritt d durchgeführt. Im vierten Verfahrensschritt d wird der im Prozessmodell PM eingesetzte, definierte Modellparameter α Verzögerungsterm α innerhalb vorgegebener Grenzwerte verändert. Die Grenzwerte für diese Veränderung des Modellparameters α werden beispielsweise durch die im Bioprozess verwendete Biomasse X vorgegeben. Im vorliegenden Beispiel für das erfindungsgemäße Verfahren wird als Modellparameter α ein Verzögerungsterm α - ein so genannter "Lag term" - eingesetzt und für den Bioprozess bzw. die Fermentation als Biomasse X Backhefe bzw. der bioaktive Organismus S. cerevisiae verwendet. Diese gibt damit auch die Grenzwerte für die Variation des Modellparameters α bzw. Verzögerungsterms α vor. Das bedeutet, im vierten Verfahrensschritt d wird der Modellparameter α bzw. der Verzögerungsterm α des Prozessmodells PM von einem Anfangswert α₀ auf einen neuen Wert α₁ geändert, wobei der neue Wert α₁ für den Verzögerungsterm innerhalb der vorgegebenen Grenzwerte liegen muss.

In der Folge werden der zweite und der dritte Verfahrensschritt b, c nochmals durchlaufen, wobei der neue Schätzwert RQ_{c} des gewählten Prozessparameter RQ für den nun aktuellen Zeitabschnitt des Bioprozesses vom Prozessmodell PM mit dem veränderten Wert α₁ des Modellparameters α bzw. Verzögerungsterms α berechnet wird. Dieser neue Schätzwert RQ_{c} wird dann in der Wiederholung des zweiten Verfahrensschritts b mit einem nun aktuellen Messwert RQₘ des gewählten Prozessparameter RQ verglichen. In der Wiederholung des dritten Verfahrensschritts c wird die auf Basis der nun aktuellen Werte für den Messwert RQₘ und den Schätzwert RQ_{c} bestimmte Abweichung ΔRQ bzw. der Betrag dieser Abweichung ΔRQ wieder mit dem vorgegebenen Schwellwert verglichen. Ist nun die Abweichung ΔRQ bzw. der Betrag der Abweichung ΔRQ kleiner als der vorgegebene Schwellwert, so wird die ursprüngliche, über dem Schwellwert liegende Abweichung ΔRQ, welche beim einem ersten bzw. davorliegenden Durchlauf des zweiten und dritten Verfahrensschritts b, c festgestellt worden ist, beispielsweise als eine biologische Variabilität oder als Variabilität in Prozessführung gewertet. Der Bioprozess bzw. die Fermentation kann damit weiterdurchgeführt werden und die Vorhersagen X_{c}, S_{c}, E_{c}, pO2_{c} der weiteren Prozessparameter X, S, E, pO2 können damit als "validiert" eingestuft werden. Das Verfahren wird dann beendet.

Wird der Schwellwert in der Wiederholung des dritten Verfahrensschritts c von der neu ermittelten Abweichung ΔRQ wieder überschritten, so kann der vierte Verfahrensschritt d erneut durchgeführt werden. D.h. der Modellparameter α bzw. der Verzögerungsterm α kann erneuert innerhalb der vorgegebenen Grenzwerte variiert werden. Der zweite bis vierte Verfahrensschritt b, c, d kann dann mit einem jeweils veränderten Modellparameter α bzw. Verzögerungsterm α für das Prozessmodell PM solange durchlaufen werden, bis die jeweils ermittelte Abweichung ΔRQ unterhalb des vorgegebenen Schwellwert gelegen ist. Bei dieser Abweichung ΔRQ können dann die Vorhersagen der weiteren Prozessparameter X, S, E, pO2 als validiert betrachtet und das Verfahren beendet werden. Die festgestellten Abweichungen ΔRQ können wieder als biologische Variabilität oder Variabilität eingestuft werden, welche den Bioprozess kaum beeinflussen.

Idealerweise werden die jeweiligen Wiederholungen des zweiten bis vierten Verfahrensschritts b, c, d mit dem jeweiligen veränderten Modellparameter α bzw. Verzögerungsterm α für das Prozessmodell PM in zeitlich geringen Abständen (z.B. 10 Sekunden) durchgeführt, um rasch zu einem validierten Prozessmodell PM bzw. zu einer qualitativen Einstufen der festgestellten Abweichungen ΔRQ zu kommen.

Für den Fall, dass nach einer vorgegebenen Anzahl von Wiederholungen des zweiten bis vierten Verfahrensschritts b, c, d mit jeweils veränderten Modellparameter α bzw. Verzögerungsterm α für das Prozessmodell PM der vorgegebene Schwellwert von der jeweils festgestellten Abweichung ΔRQ zwischen dem jeweils aktuellen Messwert RQₘ und dem entsprechenden Schätzwert RQ_{c} des gewählten Prozessparameters RQ immer noch überschritten wird, wird das Verfahren abgebrochen. Es kann dann beispielsweise eine Warnung für den Operator ausgegeben werden, um ein Eingreifen in den Bioprozess bzw. die Fermentation zu bewirken. Bei diesem Eingreifen kann der Bioprozess begutachtet und z.B. die Sensorik überprüft werden Im schlimmsten Fall muss der Bioprozess abgebrochen werden.

Im Fall, dass die Abweichung ΔRQ durch wiederholte Variation des Modellparameters α nicht behoben werden kann, wird diese Abweichung ΔRQ als "Prozessabweichung" betrachtet bzw. es wird davon ausgegangen, dass ein Fehler im Prozess wie z.B. ein Ausfall der pH-Wert-Regelung, eine fehlerhafte oder falsche Begasung, Verunreinigung im Bioreaktor, etc. vorliegt.

Die mit dem Prozessmodell PM ermittelten Schätzwerte X_{c}, S_{c}, E_{c}, pO2_{c} der weiteren Prozessparameter X, S, E, pO2 können damit als "nicht validiert" eingestuft werden. Durch Wiederholungen des zweiten bis vierten Verfahrensschritts b, c, d mit dem jeweiligen veränderten Modellparameter α bzw. Verzögerungsterm α für das Prozessmodell PM in zeitlich geringen Abständen (z.B. 10 Sekunden) kann in diesem Fall zeitnah und rasch reagiert werden. Mögliche Schäden an Bioprozess und/oder Equipment kann damit vermieden werden.

Insgesamt bietet das erfindungsgemäße Verfahren den Vorteil, dass eine Anzahl an Bioprozessen, welche sonst im Zweifelsfall abgebrochen werden, verringert wird, dass eine auftretende Abweichung ΔRQ zwischen dem jeweils aktuellen Messwert RQₘ und dem entsprechenden Schätzwert RQ_{c} des gewählten Prozessparameters RQ bewertet und qualitativ eingestuft werden können. Dadurch können die Effizienz im Einsatz an Ressourcen und Rohstoffen gesteigert und die Betriebskosten minimiert werden.

## Patentansprüche

1. Verfahren zur Überwachung von Bioprozessen, insbesondere von Fermentationsprozessen, wobei ein Verlauf eines Bioprozesses mit Hilfe eines Prozessmodells (PM) vorhergesagt und mit dem Prozessmodell (PM) während des Verlaufs des Bioprozesses Schätzwerte für biologische Prozessparameter abgeschätzt werden, mit folgenden Schritten:
a. Auswählen von zumindest dem so genannten Respiratorischen Quotienten (RQ) als einem Prozessparameter des Bioprozesses, für welchen während des Verlaufs des Bioprozesses aktuelle Messwerte (RQₘ)bestimmt werden;
b. Vergleichen des jeweils aktuellen Messwerts (RQₘ) des zumindest einen ausgewählten Prozessparameters (RQ) mit dem entsprechenden vom Prozessmodell (PM) abgeschätzten Schätzwert (RQ_{c}) für diesen zumindest einen Prozessparameter (RQ);
c. Vergleichen einer Abweichung (ΔRQ) zwischen dem jeweils aktuellen Messwert (RQₘ) und dem entsprechenden Schätzwert (RQ_{c}) für den zumindest einen ausgewählten Prozessparameter (RQ) mit einem vorgegebenen Schwellwert;
d. Verändern zumindest des so genannten Verzögerungsterms (α), welcher im Prozessmodell (PM) als definierter Modellparameter (α) eingesetzt wird, bei Überschreiten des vorgegebenen Schwellwerts durch die Abweichung (ΔRQ),
wobei die Schritte b bis d mit dem jeweils veränderten Modellparameter (α) solange durchlaufen werden, bis die Abweichung (ΔRQ) unterhalb vorgegebenen Schwellwert gelegen ist, wobei der so genannte Verzögerungsterm (α), welcher als Modellparameter (α) im Prozessmodell (PM) eingesetzt wird, bei Überschreiten des Schwellwerts durch die Abweichung (ΔRQ) im Schritt d innerhalb vorgegebener Grenzwerte verändert wird, und wobei im Fall, dass nach einer vorgegebenen Anzahl von Wiederholungen der Schritte b bis d der Schwellwert von der Abweichung (ΔRQ) nicht unterschritten wird, das Verfahren abgebrochen und Warnung ausgegeben wird.

2. Verfahren nach Anspruch 1, ***dadurch gekennzeichnet, dass*** die Grenzwerte für ein Verändern des Verzögerungsterms (α) durch eine im jeweiligen Bioprozess eingesetzte Biomasse (X), insbesondere bioaktive Zellen oder Organismen, vorgegeben werden.

3. Verfahren nach einem der vorangegangenen Ansprüche, ***dadurch gekennzeichnet, dass*** als zu messender Prozessparameter (RQₘ) ein Prozessparameter (RQ) des Bioprozesses ausgewählt wird, für welchen während des Verlaufs des Bioprozesses annähernd in Echtzeit Messwerte bestimmt werden können.

4. Verfahren nach einem der vorangegangenen Ansprüche, ***dadurch gekennzeichnet, dass*** die jeweiligen Wiederholungen der Schritte b bis d mit dem jeweils veränderten Modellparameter (α) in zeitlich geringen Abständen zueinander durchgeführt werden.

5. Verfahren nach einem der vorangegangenen Ansprüche, ***dadurch gekennzeichnet, dass*** als Prozessparameter (RQ), welcher während des Verlaufs des Bioprozesses gemessen wird, der so genannte respiratorische Quotient (RQ) und/oder eine Konzentration der Biomasse (X) und/oder des Substrats (S) ausgewählt wird.

6. Verfahren nach einem der vorangegangenen Ansprüche, ***dadurch gekennzeichnet, dass*** zum Abschätzen der Prozessparameter des Bioprozesses ein so genanntes deterministisches Prozessmodell (PM) eingesetzt wird.

## Claims

1. Method of monitoring bioprocesses, in particular fermentation processes, wherein a course of a bioprocess is predicted with the aid of a process model (PM) and estimated values for biological process parameters are estimated during the course of the bioprocess with the process model (PM), with the following steps:
a. selecting at least what is known as the respiratory quotient (RQ) as a process parameter of the bioprocess, for which current measured values (RQₘ) are determined during the course of the bioprocess;
b. comparing the respective current measured value (RQₘ) of the at least one selected process parameter (RQ) with the corresponding estimated value (RQ_{c}) estimated by the process model (PM) for this at least one process parameter (RQ);
c. comparing a variance (ΔRQ) between the respective current measured value (RQₘ) and the corresponding estimated value (RQ_{c}) for the at least one selected process parameter (RQ) with a predetermined threshold value;
d. changing at least what is known as the lag term (α), which is employed as a defined model parameter (α) in the process model (PM), when the predetermined threshold value is exceeded by the variance (ΔRQ),
wherein the steps b to d with the respective changed model parameter (α) are run through until the variance (ΔRQ) is placed below a predetermined threshold value, wherein what is known as the lag term (α), which is employed as a model parameter (α) in the process model (PM), is changed when the threshold value is exceeded within predetermined limit values by the variance (ΔRQ) in the step d and wherein in the case that after a predetermined number of repetitions of steps b to d, the threshold value is met by the variance (ΔRQ), the method is discontinued and a warning is output.

2. Method according to claim 1, ***characterised in that*** the limit values for changing the lag term (α) are predetermined by a biomass (X) employed in the respective bioprocess, in particular bioactive cells or organisms.

3. Method according to one of the preceding claims, ***characterised in that*** a process parameter (RQ) of the bioprocess is selected as a process parameter (RQₘ) to be measured, for which during the course of the bioprocess measured values can be determined approximately in real time.

4. Method according to one of the preceding claims, ***characterised in that*** the respective repetitions of steps b to d are carried out with the respective changed model parameter (α) at short intervals in time in relation to one another.

5. Method according to one of the preceding claims, ***characterised in that*** what is known as the respiratory quotient (RQ) and/or a concentration of the biomass (X) and/or of the substrate (S) is selected as a process parameter (RQ) which is measured during the course of the bioprocess.

6. Method according to one of the preceding claims, ***characterised in that*** what is known as a deterministic process model (PM) is employed to estimate the process parameters of the bioprocess.

## Revendications

1. Procédé pour la surveillance de bioprocessus, en particulier de processus de fermentation, dans lequel un déroulement d'un bioprocessus est prédit à l'aide d'un modèle de processus (PM) et des valeurs d'estimation pour des paramètres de processus biologiques sont estimées avec le modèle de processus (PM) pendant le déroulement du bioprocessus, avec les étapes suivantes :
a. sélection d'au moins le dit quotient respiratoire (RQ) en tant que paramètre de processus du bioprocessus pour lequel des valeurs de mesure réelles (RQₘ) sont déterminées pendant le déroulement du bioprocessus ;
b. comparaison de la valeur de mesure réelle (RQₘ) respective de l'au moins un paramètre de processus (RQ) sélectionné avec la valeur d'estimation (RQ_{c}) estimée par le modèle de processus (PM) correspondante pour cet au moins un paramètre de processus (RQ) ;
c. comparaison d'un écart (ΔRQ) entre la valeur de mesure réelle (RQₘ) respective et la valeur d'estimation (RQ_{c}) correspondante pour l'au moins un paramètre de processus (RQ) sélectionné avec une valeur de seuil prédéfinie ;
d. modification d'au moins le dit terme de temporisation (α) qui est utilisé dans le modèle de processus (PM) en tant que paramètre de modèle (α) défini lors du dépassement de la valeur de seuil prédéfinie par l'écart (ΔRQ),
dans lequel les étapes b à d sont soumises au paramètre de modèle (α) modifié respectivement jusqu'à ce que l'écart (ΔRQ) soit situé au-dessous de la valeur de seuil prédéfinie, dans lequel le dit terme de temporisation (α) qui est utilisé en tant que paramètre de modèle (α) dans le modèle de processus (PM) est modifié lors du dépassement de la valeur de seuil par l'écart (ΔRQ) à l'étape d à l'intérieur de valeurs limites prédéfinies, et dans lequel, dans le cas où après un nombre prédéfini de répétitions des étapes b à d la valeur de seuil n'est pas dépassée par l'écart (ΔRQ), le procédé est interrompu et une alerte est émise.

2. Procédé selon la revendication 1, **caractérisé en ce que** les valeurs limites pour une modification du terme de temporisation (α) sont prédéfinies par une biomasse (X) utilisée dans le bioprocessus respectif, en particulier des cellules ou organismes bioactifs.

3. Procédé selon l'une des revendications précédentes, **caractérisé en ce qu'**est sélectionné en tant que paramètre de processus à mesurer (RQₘ) un paramètre de processus (RQ) du bioprocessus pour lequel des valeurs de mesure peuvent être déterminées presque en temps réel pendant le déroulement du bioprocessus.

4. Procédé selon l'une des revendications précédentes, **caractérisé en ce que** les répétitions respectives des étapes b à d sont mises en oeuvre dans des intervalles de temps faibles les unes par rapport aux autres avec le paramètre de modèle (α) modifié respectivement.

5. Procédé selon l'une des revendications précédentes, **caractérisé en ce que** sont sélectionnés en tant que paramètre de processus (RQ) qui est mesuré pendant le déroulement du bioprocessus le dit quotient respiratoire (RQ) et/ou une concentration de la biomasse (X) et/ou du substrat (S).

6. Procédé selon l'une des revendications précédentes, **caractérisé en ce que**, pour l'estimation des paramètres de processus du bioprocessus, un dit modèle de processus (PM) déterministe est utilisé.
